# EUROPÄISCHE PATENTSCHRIFT

(11) **EP 1 896 021 B1**
(45) Veröffentlichungstag und Bekanntmachung des Hinweises auf die Patenterteilung: **15.04.2009**
(21) Anmeldenummer: 06754265.4
(22) Anmeldetag: 09.06.2006
(51) Int. Cl.: A61K 31/4433, C07D 405/12, A61K 9/20, A61P 25/16

(54) **FESTE PHARMAZEUTISCHE ZUBEREITUNG ENTHALTEND (R)-(-)-2-[5-(4-FLUOROPHENYL)-3-PYRIDYLMETHYLAMINOMETHYL]-CHROMAN**
SOLID PHARMACEUTICAL PREPARATION CONTAINING (R)-(-)-2-[5-(4-FLUOROPHENYL)-3-PYRIDYLMETHYLAMINOMETHYL]-CHROMAN
PREPARATION PHARMACEUTIQUE SOLIDE CONTENANT DU (R)-(-)-2-[5-(4-FLUOROPHENYL)-3-PYRIDYLMETHYLAMINOMETHYL]-CHROMANE

(30) Priorität: 21.06.2005 EP 05013293
(43) Veröffentlichungstag der Anmeldung: 12.03.2008
(73) Patentinhaber: Merck Patent GmbH, 64293 Darmstadt (DE)
(72) Erfinder: KUNATH, Klaus, 63165 Muehlheim (DE); HEIL, Kirstin, 64291 Darmstadt (DE); RUPP, Roland, 51467 Bergisch-Gladbach (DE)
(86) Internationale Anmeldenummer: PCT/EP2006/005563
(87) Internationale Veröffentlichungsnummer: WO 2006/136302

(56) Entgegenhaltungen:
- EP-A- 1 192 942
- WO-A-01/68063

## Beschreibung

Die vorliegende Erfindung betrifft eine feste pharmazeutische Zubereitung enthaltend (R)-(-)-2-[5-(4-Fluorophenyl)-3-pyridylmethylaminomethyl]-chroman als Wirkstoff sowie die Herstellung der festen pharmazeutischen Zubereitung.

(R)-(-)-2-[5-(4-Fluorophenyl)-3-pyridylmethylaminomethyl]-chroman ist der erste Vertreter einer neuen Klasse von atypischen Neuroleptika, die kombiniert sowohl zu einer selektiven Blockade von Dopamin D₂-Rezeptoren (D₂-Antagonist) als auch selektiven Stimulierung von Serotonin 5-HT_{1A}-Rezeptoren (5-HT_{1A}-Agonist) führen. Auf Grund des neuartigen Wirkprinzips kann (R)-(-)-2-[5-(4-Fluorophenyl)-3-pyridylmethylaminomethyl]-chroman zur Behandlung der Schizophrenie und zur Behandlung der Dyskinesie/Psychose in der Parkinsonschen Krankheit eingesetzt werden. Klinisch entwickelt werden soll (R)-(-)-2-[5-(4-Fluorophenyl)-3-pyridylmethylaminomethyl]-chroman zur Behandlung der Dyskinesie in der Parkinsonschen Krankheit.

Die klinische Entwicklung und anschließende Vermarktung dieses Wirkstoffes erfordert eine leicht verabreichbare, vorzugsweise eine feste, oral zu verabreichende pharmazeutische Zubereitung. Die pharmazeutische Zubereitung sollte keine toxikologisch bedenklichen Hilfsstoffe enthalten, eine gute Wirkstofffreisetzung gewährleisten, und über längere Zeit auch bei erhöhter Temperatur und Luftfeuchtigkeit stabil lagerfähig sein.

Es wurden umfangreiche Versuche durchgeführt, um eine diesen Anforderungen entsprechende feste pharmazeutische Zubereitung zur Verfügung zustellen. Die Versuche ergaben jedoch keine diesen Anforderungen entsprechende feste pharmazeutische Zubereitung, insbesondere ergab sich wiederholt eine mangelnde Stabilität des Wirkstoffes (R)-(-)-2-[5-(4-Fluorophenyl)-3-pyridytmethylaminomethyl]-chroman.

WO01/68063 offenbart verschiedene pharmazeutische Zubereitungen von (R)-(-)-2-[5-(4-Fluorophenyl)-3-pyridylmethylaminomethyl]chroman.

EP-A-1192942 offenbart pharmazeutische Zusammensetzungen auf der Basis von niedersubstituierter Hydroxypropylzellulose und Zuckeralkoholen.

Es war Aufgabe der vorliegenden Erfindung eine feste, oral zu verabreichende Formulierung für (R)-(-)-2-[5-(4-Fluorophenyl)-3-pyridylmethylaminomethyl]-chroman zur Verfügung zu stellen, die den oben genannten Anforderungen entspricht. Die Zubereitung sollte insbesondere eine den Anforderungen entsprechende Wirkstofffreisetzung gewährleisten, keine toxikologisch bedenklichen Hilfsstoffe enthalten, und in Klimazone I-IV, d.h. auch unter erhöhter Temperatur und Luftfeuchtigkeit, über längere Zeit stabil lagerfähig sein.

Überraschenderweise konnte eine diesen Anforderungen entsprechende Zubereitung zur Verfügung gestellt werden, wenn diese neben dem Wirkstoff einen oder mehrere Zuckeralkohol/e als Füllstoff/e enthält. Gegenstand der vorliegenden Erfindung ist daher eine feste pharmazeutische Zubereitung enthaltend (R)-(-)-2-[5-(4-Fluorophenyl)-3-pyridylmethylaminomethyl]-chroman als Wirkstoff und mindestens einen Zuckeralkohol als Füllstoff.

(R)-(-)-2-[5-(4-Fluorophenyl)-3-pyridylmethylaminomethyl]-chroman kann in der pharmazeutischen Zubereitung als freie Base oder in Form eines seiner Säureadditionssalze wie z. B. als Hydrobromid, Hydrochlorid, Dihydrochlorid, Acetat, Aspartat, Benzoat, Citrat, Fumarat, Glutamat, Maleat, Methansulfonat oder Tartrat enthalten sein. Bevorzugt ist (R)-(-)-2-[5-(4-Fluorophenyl)-3-pyridylmethylaminomethyl]-chroman als Säureadditionssalz, besonders bevorzugt als Hydrochlorid-Salz, enthalten.

Die erfindungsgemäße feste pharmazeutischen Zubereitung enthält einen oder mehrere Zuckeralkohol/e. Unter Zuckeralkohole werden Monosaccharide verstanden, deren reaktive Carbonylgruppe zur Alkoholgruppe reduziert ist, wie z. B. Hexite oder Pentite. Bevorzugt enthält die erfindungsgemäße feste Zubereitung als Zuckeralkohol/e Hexite wie z. B. Mannitol, Sorbitol, Dulcitol, Xylitol oder Ribitol. Besonders bevorzugt sind Mannitol und/oder Sorbitol, ganz besonders bevorzugt ist Mannitol enthalten.

Der/die Zuckeralkohol/e können in allen ihren Modifikationen eingesetzt sein, in denen diese vorkommen. Wird z. B. Mannitol als Zuckeralkohol eingesetzt, kann dieser in Form einer oder mehrerer seiner polymorphen Formen, z.B. der α-, β-, γ- oder δ-Modifikation, vorliegen. Bevorzugt wird Mannitol in Form seiner β- und/oder δ-Modifikation eingesetzt, besonders bevorzugt in Form seiner δ-Modifikation, d. h. als δ-Mannitol. Die Verwendung von δ-Mannitol ist insbesondere dann bevorzugt, wenn die ' Zubereitung während ihrer Herstellung mit Wasser versetzt wird, wie dies beispielsweise bei Feuchtgranulierung erfolgt. Hierbei wandelt sich ein Teil des δ-Mannitols zu β-Mannitol um, und durch diese Umwandlung verbessern sich die Tablettiereigenschaften.

Auf Grund ihrer Herstellung durch Reduktion von Monosacchariden können Zuckeralkohole infolge unvollständiger Umsetzung der Carbonylgruppen noch geringe Anteile an Zuckern enthalten, die reduzierend wirken können. Vorzugsweise enthält die erfindungsgemäße Zubereitung daher Zuckeralkohole in einer Qualität, die einen möglichst geringen Anteil an solchen nicht oder unvollständig umgesetzten Zucker enthält. Bevorzugt ist daher, dass die erfindungsgemäße Zubereitung Zuckeralkohol/e enthält, der/die weniger als 0,2 Gew.-%, besonders bevorzugt weniger als 0,05 Gew.-% reduzierende Zucker, enthält/enthalten.

Die feste pharmazeutische Zubereitung kann als Pulver, Granulat, Pellets, Kapsel oder Tablette vorlieben. Während Kapseln und Tabletten die jeweils zur Einnahme vorgesehenen Menge an Wirkstoff als klar definierte Einzeldosis zur Verfügung stellen, kann mittels Pulvern, Pellets und Granulaten die jeweils erforderliche Wirkstoffmenge in einfacher Weise angepasst werden. Granulate sind rieselfähige körnige Aggregate von Pulvern die durch Granulierung hergestellt werden können. Pellets sind feste kleine kugelförmige Arzneiformen, wie z. B. Granulatkörner oder Mikrotabletten, mit einem sehr engen Korngrößenbereich. Granulate und Pellets stellen eine eigenständige Arzneiform dar, können aber auch als . Zwischenprodukt für die Herstellung von Tabletten dienen. Sollen vorbestimmte Wirkstoffmengen mittels Pulvern, Granulaten oder Pellets verabreicht werden können diese zur Sicherstellung einer ausreichenden Dosierungsgenauigkeit auch als abgeteilte Pulver/Granulate oder in Kapseln abgefüllt bereitgestellt werden. Bevorzugt liegt die erfindungsgemäße pharmazeutische Zubereitung als Granulat, Kapsel oder Tablette, besonders bevorzugt als Kapsel oder Tablette, ganz besonders bevorzugt als Tablette vor.

Je nach Arzneiform kann die erfindungsgemäße feste Zubereitung unterschiedliche Hilfsstoffe wie z. B. Bindemittel, zusätzliche Füllstoffe, Sprengmittel, Fließregulierungsmittel oder Gleitmittel enthalten.

Bindemittel werden insbesondere als Hilfsstoffe für die Herstellung von Granulaten und aus Granulaten hergestellten Kapseln und Tabletten eingesetzt und sind u. a. für den Zusammenhalt der Pulverpartikel im Granulatskorn verantwortlich. Einsetzbare Bindemittel sind zum Beispiel Polymere wie zum Beispiel Polyvinylpyrrolidon oder Polyvinylacetat, Stärkekleister wie z. B. Maisstärkekleister, Cellulosederivate wie z. B. Hydroxypropylmethylcellulose oder Hydroxypropylcellulose. Bevorzugt enthält die erfindungsgemäße feste Zubereitung als Bindemittel Cellulosederivate, wobei Hydroxypropylmethylcellulose besonders bevorzugt ist. Je nach Art des Bindemittels kann dieses in der erfindungsgemäßen festen Zubereitung in einem Anteil von 0,1 bis 80 Gew.-% enthalten sein. Bevorzugt enthält die erfindungsgemäße feste Zubereitung 1 bis 5 Gew.-%, besonders bevorzugt 1,5 bis 3 Gew.-% Bindemittel.

Sprengmittel können enthalten sein, um die Zerfallszeit von Tabletten zu verkürzen, so dass der Wirkstoff aus den Tabletten rasch freigesetzt wird. Beispiele für erfindungsgemäß einsetzbare Sprengmittel sind mikrokristalline Cellulose, quer vernetztes Polyvinylpyrrolidon wie z. B. Crosspovidone oder vernetzte Carboxymethylcellulose. Besonders bevorzugt enthält die erfindungsgemäße feste Zubereitung Carboxymethylcellulose, ganz besonders bevorzugt quer vernetzte Carboxymethylcellulose, als Sprengmittel. Je nach Art des Sprengmittels kann dieses in der erfindungsgemäßen festen Zubereitung in einem Gewichtsanteil von 0,01 bis 20 Gew.-% enthalten sein. Bevorzugt enthält die erfindungsgemäße feste Zubereitung 0,1 bis 5 Gew.-%, besonders bevorzugt 0,2 bis 2 Gew.% an Sprengmittel.

Fließregulierungsmittel können in Pulvern oder Granulaten enthalten sein und werden diesen beigemischt, um ihre Rieselfähigkeit zu erhöhen. Ebenso können Fließregulierungsmittel in Tabletten enthalten sein, soweit diese durch Verpressung von Pulvern oder Granulaten hergestellt werden. Auch in diesem Fall werden sie den Pulvern/Granulaten beigemischt, um deren Rieselfähigkeit zu erhöhen, insbesondere um eine gleichmäßige Füllung der Matrizen vor der Verpressung zur Tablette und damit eine hohe Dosierungsgenauigkeit sicherzustellen. Als Fließregulierungsmittel einsetzbar sind zum Beispiel hochdisperses Siliziumdioxid (Aerosil) oder getrocknete Stärke. Bevorzugt enthält die erfindungsgemäße feste Zubereitung hochdisperses Siliziumdioxid als Fließregulierungsmittel. Fließregulierungsmittel sind in der erfindungsgemäßen festen Zubereitung vorzugsweise in einem Anteil von 0,1 bis 3 Gew.-% enthalten, bevorzugt sind 0,2 bis 2 Gew.-%, besonders bevorzugt sind 0,3 bis 1 Gew.-%.

Soweit die erfindungsgemäße feste Zubereitung eine Tablette ist, kann diese auch Schmiermittel enthalten, um während des Tablettiervorgangs die Gleitreibung des Tablettierguts und des Pressstempels in der Matrize herabzusetzen und ein Kleben an den Stempeln zu vermeiden. Geeignete Schmiermittel sind Erdalkalimetall-Salze von Fettsäuren wie zum Beispiel Magnesiumstearat, höhere Fettalkohole oder Talkum. Bevorzugt enthält die erfindungsgemäße feste Zubereitung Magnesiumstearat als Schmiermittel. Schmiermittel sind in der erfindungsgemäßen festen Zubereitung vorzugsweise in einem Anteil von 0,1 bis 5 Gew.-% enthalten, bevorzugt sind 0,5 bis 5 Gew.-%, besonders bevorzugt 1 bis 3 Gew.-%.

Ist die erfindungsgemäße feste Zubereitung eine Tablette, kann diese mit einem Überzug versehen sein. Als Überzug eignen sich filmbildende Polymere wie z. B. solche aus der Gruppe der Cellulosederivate, Dextrine, Stärken, natürlichen Gummen, wie z. B. Gummi Arabicum, Xanthane, Alginate, Polyvinylalcohol, Polymethacrylate und dessen Derivate wie z. B. Eudragite, die mittels der verschiedenen pharmazeutischen üblichen Verfahren, wie z. B. Film-Coating, als Lösungen oder Suspensionen auf die Tablette aufgetragen werden können. Üblicherweise werden hierbei Lösungen/Suspensionen verwendet, die neben dem filmbildenden Polymeren noch weitere Hilfsstoffe wie Hydrophilisatoren, Weichmacher, Tenside, Farbstoffe und Weißpigmente, wie z. B. Titandioxid enthalten.

Die Herstellung der erfindungsgemäßen festen Zubereitung kann gemäß den dem Fachmann bekannten Verfahren erfolgen.

Die Herstellung von Pulvern kann beispielsweise erfolgen indem der Wirkstoff mit dem Zuckeralkohol sowie gegebenenfalls mit weiteren Hilfsmitteln wie Fließregulierungsmitteln versetzt und anschließend gemischt wird.

Die Herstellung der Granulate erfolgt durch Granulierung, die grundsätzlich auf feuchtem oder trockenem Weg erfolgen kann. Bei Feuchtgranulierung wird beispielsweise eine Pulvermischung enthaltend den Wirkstoff zusammen mit dem Zuckeralkohol sowie gegebenenfalls weiteren geeigneten Hilfsstoffen, mit einer Granulierflüssigkeit, die vorzugsweise ein Bindemittel enthält, versetzt, in die Aggregate geeigneter Größe (Granulate) überführt und anschließend getrocknet. Der Wirkstoff kann auch durch Suspension in der Granulierflüssigkeit in das Granulat eingebracht werden. Die Überführung der Pulvermischung in Aggregate geeigneter Größe kann z. B. durch die so genannte Aufbaugranulierung, beispielsweise im Dragierkessel, mittels Tellergranulierung oder im Wirbelschichtverfahren, z. B. nach dem Glatt- oder Wurster-Verfahren, oder durch die so genannte Abbaugranulierung erfolgen, in der die Pulvermischung zunächst befeuchtet und zu einer plastisch verformbaren Masse verarbeitet und anschließend, beispielsweise durch Extrusion durch ein Sieb mit Maschen geeigneter Größe, in die Aggregate der gewünschten Größe überführt wird. Bei Trockengranulierung wird die Pulvermischung beispielsweise mittels Kompaktieren zwischen zwei gegenläufig rotierenden Kompaktierwalzen zu Schülpen verpresst, die anschließend zu Granulaten zerkleinert werden.

Pellets können durch Granulieren und anschließendem Abrunden (Sphäronisieren), beispielsweise mittels Tellergranulierung, oder auch durch Verpressung von Pulvern oder Granulaten zu Mikrotabletten hergestellt werden.

Die Herstellung der erfindungsgemäßen Zubereitung in Form von Tabletten kann durch Verpressen von Pulvermischungen (Direkttablettierung) oder durch Verpressen von Granulaten erfolgen. Im einfachsten Fall der Direkttablettierung wird zunächst der Wirkstoff mit dem Zuckeralkohol (in einer direkt tablettierbaren Qualität) sowie gegebenenfalls weiteren Hilfsstoffen vermischt und die erhaltenen Pulvermischung direkt zu der erfindungsgemäßen festen Zubereitung verpresst.

Wird die erfindungsgemäße Zubereitung in Form einer Tablette durch Verpressung von Granulaten hergestellt, können hierzu Granulate eingesetzt werden, die durch Feucht- oder Trockengranulierung hergestellt wurden. Vorteilhaft werden die Granulate vor ihrer Verpressung noch mit einer so genannten äußeren Phase enthaltend einen Hilfsstoff oder eine Mischung aus mehreren Hilfsstoffen, insbesondere Schmiermittel, Fließregulierungsmittel und/oder Sprengmittel, vermischt.

Wird die erfindungsgemäße Zubereitung in Form einer Tablette durch Verpressung von mittels Feuchtgranulierung hergestelltem Granulat hergestellt und enthält das Granulat als Zuckeralkohol Mannitol, wird dieses vorzugsweise in Form seine δ-Modifikation, d. h. als δ-Mannitol eingesetzt, denn δ-Mannitol wird während der Feuchtgranulierung zumindest teilweise in β-Mannitol umgewandelt. Hierdurch vergrößert sich die Oberfläche, was vorteilhaft zu deutlich härteren Tabletten führt, als wenn direkt β-Mannitol eingesetzt wird.

Nach einer bevorzugten Ausführungsform der Erfindung ist die feste Zubereitung eine Tablette und enthält 0,1 bis 10 Gew.-% (R)-(-)-2-[5-(4-Fluorophenyl)-3-pyridylmethylaminomethyl]-chroman, 50 bis 99,9 Gew.-% Mannitol und 1 bis 5 Gew.-% Hydroxypropylmethylcellulose.

Nach einer besonders vorteilhaften Ausführungsform ist die feste Zubereitung eine Tablette und enthält 0,2 bis 2 Gew.-% (R)-(-)-2-[5-(4-Fluorophenyl)-3-pyridylmethylaminomethyl]-chroman, 90 bis 98 Gew.-% Mannitol und 1,5 bis 3 Gew.-% Hydroxypropylmethylcellulose.

Gegenstand der vorliegenden Patentanmeldung ist weiterhin ein Verfahren zur Herstellung einer festen pharmazeutischen Zubereitung in Form einer Tablette, das dadurch gekennzeichnet ist, dass diese durch Direkttablettierung oder durch Verpressung von mittels Feucht- oder Trockengranulierung hergestellten Granulaten hergestellt und anschließend gegebenenfalls mit einem Überzug versehen wird.

Die Ausführungsbeispiele, ohne hierauf beschränkt zu sein, erläutern die Erfindung.

### Beispiel 1

Pulvermischung enthaltend
1,0 mg (R)-(-)-2-[5-(4-Fluorophenyl)-3-pyridylmethylaminomethyl]-chroman
199 mg D-Mannitol

Die Herstellung der Pulvermischung erfolgt durch Mischen des Wirkstoffes mit Mannitol.

### Beispiel 2

Granulat enthaltend
1,0 mg (R)-(-)-2-[5-(4-Fluorophenyl)-3-pyridylmethylaminomethyl]-chroman
214,74 mg D-Mannitol
5,06 mg Hydroxypropylmethylcellulose

Die Granulate werden hergestellt durch Granulation des Wirkstoffes mit Mannitol und HPMC-Lösung in der Wirbelschicht. Der Wirkstoff wird dazu in der HPMC-Lösung suspendiert und auf das Mannitol aufgesprüht.

### Beispiel 3

Tablette (Batch 010708) enthaltend
1,0 mg (R)-(-)-2-[5-(4-Fluorophenyl)-3-pyridylmethylaminomethyl]-chroman
214,74 mg D-Mannitol
5,06 mg Hydroxypropylmethylcellulose
2,30 mg Croscarmellose-Na
1,15 mg Hochdisperses Siliciumdioxid
5,75 mg Magnesiumstearat

Die Tabletten werden hergestellt durch Granulation des Wirkstoffes mit Mannitol und HPMC-Lösung in der Wirbelschicht. Der Wirkstoff wird dazu in der HPMC-Lösung suspendiert und auf das Mannitol aufgesprüht. Dem Granulat werden Croscarmellose, Siliciumdioxid und Magnesiumstearat zugemischt, die erhaltene Mischung wird zu Tabletten verpresst.
Die Tabletten werden in HDPE-Flaschen gefüllt, über vorbestimmte Zeiten unter definierten Klimabedingungen eingelagert und anschließend im Hinblick auf Wirkstoffgehalt und Abbauprodukte untersucht. Lagerzeiten, Klimabedingungen und die jeweils gemessenen Mengen an Wirkstoff sowie Abbauprodukten sind in Tabelle 1 zusammengestellt.

**Tabelle 1**

| Wochen | Klima (°C / % r.F.) | Packmittel | Gehalt (%) | Summe aller Abbauprodukte (%) |
|---|---|---|---|---|
| 13 | 25/60 | HDPE | 99,19 | 0,10 |
| 26 | 25/60 | HDPE | 100,17 | 0,14 |
| 13 | 30/65 | HDPE | 98,71 | 0,14 |
| 26 | 30/65 | HDPE | 98,44 | 0,22 |
| 13 | 40/75 | HDPE | 97,88 | 0,32 |
| 26 | 40/75 | HDPE | 98,36 | 0,54 |

### Beispiel 4

Tablette (Batch 9344, direkttablettiert) enthaltend
1,0 mg (R)-(-)-2-[5-(4-Fluorophenyl)-3-pyridylmethylaminomethyl]-chroman
116,2 mg Mannitol
1 mg Croscarmellose
1,8 mg Magnesiumstearat

Die Tablettenbestandteile werden miteinander gemischt, zu Tabletten verpresst und in Gläser abgefüllt. Die verschlossenen Gläser werden über vorbestimmte Zeiten unter definierten Klimabedingungen eingelagert und anschließend im Hinblick auf Wirkstoffgehalt und Abbauprodukte untersucht. Lagerzeiten, Klimabedingungen und die jeweils gemessenen Mengen an Wirkstoff sowie Abbauprodukten sind in Tabelle 2 zusammengestellt.

**Tabelle 2**

| Wochen | Klima (°C / % r.F.) | Packmittel | Gehalt (%) | Summe aller Abbauprodukte (%) |
|---|---|---|---|---|
| 22 | 25/60 | Glas | 94,3 | 0,14 |
| 22 | 30/60 | Glas | 93,8 | 0,17 |
| 22 | 40/75 | Glas | 95,3 | 0,22 |

### Beispiel 5

Kapsel (Batch 9047) enthaltend
0,5 mg (R)-(-)-2-[5-(4-Fluorophenyl)-3-pyridylmethylaminomethyl]-chroman
126,5 mg Mannitol
2,0 mg Hydroxypropylmethylcellulose
2,0 mg Magnesiumstearat
Hartgelatinekapsel Größe 4

Der Wirkstoff wird zusammen mit Mannitol mit einer HPMC-Lösung granuliert. Das erhaltene Granulat wird mit Magnesiumstearat vermischt, in Hartgelatinekapseln abgefüllt und in Gläser überführt. Die verschlossenen Gläser werden über vorbestimmte Zeiten unter definierten Klimabedingungen eingelagert und anschließend im Hinblick auf Wirkstoffgehalt und Abbauprodukte untersucht. Lagerzeiten, Klimabedingungen und die jeweils gemessenen Mengen an Wirkstoff sowie Abbauprodukten sind in Tabelle 3 zusammengestellt.

**Tabelle 3**

| Wochen | Klima (°C / % r.F.) | Packmittel | Gehalt (%) | Summe aller Abbauprodukte (%) |
|---|---|---|---|---|
| 13 | 25/60 | Glas | 97,3 | 0,08 |
| 26 | 25/60 | Glas | 95,6 | 0,34 |
| 13 | 40/75 | Glas | 96,7 | 0,20 |
| 26 | 40/75 | Glas | 95,9 | 0,95 |

### Vergleichsbeispiel 1 - (Lactose-Kapsel, Batch 9314)

Kapsel enthaltend
0,500 mg (R)-(-)-2-[5-(4-Fluorophenyl)-3-pyridylmethylaminomethyl]-chroman
126,5 mg Lactose fein gepulvert
2,0 mg Hydroxypropylmethylcellulose
1,0 mg Magnesiumstearat
Hartgelatinekapsel Größe 4

Der Wirkstoff wird analog dem in Beispiel 2 beschriebenen Verfahren mit Lactose mit HPMC-Lösung granuliert. Das Granulat wird mit Magnesiumstearat als Schmiermittel vermischt und Hartgelatine-Leerkapseln abgefüllt, unter definierten Klimabedingungen eingelagert und anschließend im Hinblick auf Wirkstoffgehalt und Abbauprodukte untersucht. Die Ergebnisse sind in Tabelle 4 zusammengestellt.

**Tabelle 4**

| Wochen | Klima (°C / % r.F.) | Packmittel | Gehalt (%) | Summe aller Abbauprodukte (%) |
|---|---|---|---|---|
| 13 | 25/60 | Glas | 101,13 | 0,43 |
| 26 | 25/60 | Glas | 101,04 | 1,29 |
| 13 | 30/60 | Glas | 100,79 | 0,97 |
| 26 | 30/60 | Glas | 99,89 | 1,93 |
| 13 | 40/75 | Glas | 96,94 | 2,37 |
| 26 | 40/75 | Glas | 98,20 | 3,60 |

### Vergleichsbeispiel 2 (CaHPO₄-Filmtablette, Batch 8782)

Filmtablette enthaltend
1,0 mg (R)-(-)-2-[5-(4-Fluorophenyl)-3-pyridylmethylaminomethyl]-chroman
100,0 mg Calciumhydrogenphosphat
10,0 mg Hydroxypropylcellulose (niedrig substituiert)
6,0 mg vorverkleisterte Stärke
1,0 mg Hydroxypropylmethylcelullose
49,75 mg mikrokristalline Cellulose
1,0 mg Hochdisperses Siliciumdioxid
3,125 mg Magnesiumstearat
1,86 mg Hydroxypropylmethylcelullose
0,455 mg Macrogol 400
1,164 mg Titan(IV)-oxid
0,233 mg Talk feinst gepulvert

Die Filmtabletten werden hergestellt durch Granulation des Wirkstoffes mit Calciumhydrogenphosphat, Hydroxypropylcellulose, vorverkleisterter Stärke und HPMC-Lösung. Dem Granulat werden mikrokristalline Cellulose, hochdisperses Siliciumdioxid und Magnesiumstearat zu gemischt, die erhaltene Mischung wird zu Tabletten verpresst, mit einer Suspension von Titandioxid und Talkum in einer HPMC/PEG Lösung im Trommelcoater überzogen und in Gläser abgefüllt.
Die Filmtabletten werden nach Lagerung über vorbestimmte Zeiten unter definierten Klimabedingungen im Hinblick auf Wirkstoffgehalt und Abbauprodukte untersucht. Lagerzeiten, Klimabedingungen und die jeweils gemessenen Mengen an Wirkstoff sowie Abbauprodukten sind in Tabelle 5 zusammengestellt.

**Tabelle 5**

| Wochen | Klima (°C / % r.F.) | Packmittel | Gehalt (%) | Summe aller Abbauprodukte (%) |
|---|---|---|---|---|
| 13 | 25/60 | Glas | 105,6 | 0,43 |
| 26 | 25/60 | Glas | 103,9 | 0,80 |
| 13 | 30/60 | Glas | 105,0 | 0,43 |
| 26 | 30/60 | Glas | 104,5 | 0,86 |
| 13 | 40/75 | Glas | 105,7 | 0,55 |
| 26 | 40/75 | Glas | 101,7 | 1,37 |

### Untersuchungen zur Stabilität der Zubereitungen

Die Stabilität der erfindungsgemäßen Zubereitungen wird in Haltbarkeitsstudien überprüft. Hierzu werden die hergestellten festen Zubereitungen bei verschiedenen Temperaturen eingelagert, zu bestimmten Zeiten ausgelagert und mit geeigneten analytischen Methoden untersucht. Als Klimabedingungen werden 25°C mit einer relativen Luftfeuchtigkeit (r.F.) von 60%, 30°C mit einer r.F. von 60 oder 65% und 40°C mit einer von r.F. von 75% gewählt. Während die erstgenannte Bedingung für Lagerung bei Raumtemperatur in Klimazone I und II steht und die zweitgenannte für Lagerung bei Raumtemperatur in Klimazone III und IV, wird die letztgenannte Bedingung als Stressbedingung ausgewählt, um bei den verschiedenen Formulierungen schnell zu Unterschieden hinsichtlich der Stabilität zu gelangen. Mögliche Instabilitäten äußern sich bei (R)-(-)-2-[5-(4-Fluorophenyl)-3-pyridylmethylaminomethyl]-chroman vornehmlich in der Bildung von Abbauprodukten.

### Analytische Testmethoden:

Identität, Reinheit und der Gehalt der festen Zubereitung enthaltend (R)-(-)-2-[5-(4-Fluorophenyl)-3-pyridylmethylaminomethyl]-chroman, werden durch Hochleistungsflüssigchromatographie mit UV-Detektion unter Verwendung einer RP-18-Säule im Hochdruckgradientensystem nach Herstellung und im Zuge der Stabilitätsstudien geprüft. Als Extraktionsmedium und mobile Phase werden Gemische aus Aceconitril und Phosphatpuffer verwendet.

## Patentansprüche

1. Feste pharmazeutische Zubereitung enthaltend (R)-(-)-2-[5-(4-Fluorophenyl)-3-pyridylmethylaminomethyl]-chroman als freie Base oder in Form eines seiner Säureadditionssalze als Wirkstoff und mindestens einen Zuckeralkohol als Füllstoff.

2. Feste pharmazeutische Zubereitung nach Anspruch 1, **dadurch gekennzeichnet, dass** (R)-(-)-2-[5-(4-Fluorophenyl)-3-pyridylmethylaminomethyl]-chroman als Hydrochloridsalz enthalten ist.

3. Feste pharmazeutische Zubereitung nach Anspruch 1 und/oder 2, **dadurch gekennzeichnet, dass** als Zuckeralkohol Sorbit und/oder Mannitol enthalten wird.

4. Feste pharmazeutische Zubereitung nach Anspruch 3, **dadurch gekennzeichnet, dass** Mannitol enthalten ist.

5. Feste pharmazeutische Zubereitung nach Anspruch 4, **dadurch gekennzeichnet, dass** Mannitol als δ-Mannitol enthalten ist.

6. Feste pharmazeutische Zubereitung nach einem oder mehreren der Ansprüche 1 bis 5, **dadurch gekennzeichnet, dass** der/die Zuckeralkohol/e weniger als 0,2 Gew.-% reduzierende Zucker enthält.

7. Feste pharmazeutische Zubereitung nach Anspruch 6, **dadurch gekennzeichnet, dass** das der/die Zuckeralkohol/e weniger als 0,05 Gew.-% reduzierende Zucker enthält.

8. Feste pharmazeutische Zubereitung nach einem oder mehreren der Ansprüche 1 bis 7, **dadurch gekennzeichnet, dass** diese als Granulat, Kapsel oder Tablette vorliegt.

9. Feste pharmazeutische Zubereitung nach Anspruch 8, **dadurch gekennzeichnet, dass** diese eine Tablette ist.

10. Feste pharmazeutische Zubereitung nach Anspruch 9 und/oder 10, **dadurch gekennzeichnet, dass** als Bindemittel Hydroxypropylmethylcellulose enthalten ist.

11. Feste pharmazeutische Zubereitung nach einem oder mehreren der Ansprüche 1 bis 10, **dadurch gekennzeichnet, dass** diese 0,1 bis 10 Gew.-% (R)-(-)-2-[5-(4-Fluorophenyl)-3-pyridylmethylaminomethyl]-chroman, 50 bis 99,9 Gew.-% Mannitol und 1 bis 5 Gew.-% Hydroxypropylmethylcellulose enthält.

12. Verfahren zur Herstellung einer festen pharmazeutischen Zubereitung gemäß einem oder mehreren der Ansprüche 1 bis 10, **dadurch gekennzeichnet, dass** diese durch Direkttablettierung oder durch Verpressung von mittels Feucht- oder Trockengranulierung hergestellten Granulaten hergestellt und anschließend gegebenenfalls mit einem Überzug versehen wird.

13. Verfahren nach Anspruch 12, **dadurch gekennzeichnet, dass** diese durch Verpressung eines mittels Feuchtgranulierung hergestellten Granulats hergestellt wird.

## Claims

1. Solid pharmaceutical composition comprising (R)-(-)-2-[5-(4-fluorophenyl)-3-pyridylmethylaminomethyl]chroman as free base or in the form of one of its acid-addition salts as active compound and at least one sugar alcohol as filler.

2. Solid pharmaceutical composition according to Claim 1, **characterised in that** (R)-(-)-2-[5-(4-fluorophenyl)-3-pyridylmethylaminomethyl]chroman is present as hydrochloride salt.

3. Solid pharmaceutical composition according to Claim 1 and/or 2, **characterised in that** the sugar alcohol present is sorbitol and/or mannitol.

4. Solid pharmaceutical composition according to Claim 3, **characterised in that** mannitol is present.

5. Solid pharmaceutical composition according to Claim 4, **characterised in that** mannitol is present in the form of δ-mannitol.

6. Solid pharmaceutical composition according to one or more of Claims 1 to 5, **characterised in that** the sugar alcohol(s) comprises (comprise) less than 0.2% by weight of reducing sugars.

7. Solid pharmaceutical composition according to Claim 6, **characterised in that** the sugar alcohol(s) comprises (comprise) less than 0.05% by weight of reducing sugars.

8. Solid pharmaceutical composition according to one or more of Claims 1 to 7, **characterised in that** it is in granule, capsule or tablet form.

9. Solid pharmaceutical composition according to Claim 8, **characterised in that** it is a tablet.

10. Solid pharmaceutical composition according to Claim 9 and/or 10, **characterised in that** the binder present is hydroxypropylmethylcellulose.

11. Solid pharmaceutical composition according to one or more of Claims 1 to 10, **characterised in that** it comprises 0.1 to 10% by weight of (R)-(-)-2-[5-(4-fluorophenyl)-3-pyridylmethylaminomethyl]chroman, 50 to 99.9% by weight of mannitol and 1 to 5% by weight of hydroxypropylmethylcellulose.

12. Process for the preparation of a solid pharmaceutical composition according to one or more of Claims 1 to 10, **characterised in that** it is prepared by direct compression or by pressing granules produced by means of moist or dry granulation, and is subsequently optionally provided with a coating.

13. Process according to Claim 12, **characterised in that** it is prepared by pressing granules produced by means of moist granulation.

## Revendications

1. Composition pharmaceutique solide comprenant du (R)-(-)-2-[5-(4-fluorophényl)-3-pyridylméthylaminométhyl]chromane sous forme de base libre ou sous forme de l'un de ses sels d'addition d'acide comme composé actif et au moins un alcool de sucre comme charge.

2. Composition pharmaceutique solide selon la revendication 1, **caractérisée en ce que** le (R)-(-)-2-[5-(4-fluorophényl)-3-pyridylméthylaminométhyl]-chromane est présent sous forme de sel de chlorhydrate.

3. Composition pharmaceutique solide selon la revendication 1 et/ou 2, **caractérisée en ce que** l'alcool de sucre présent est le sorbitol et/ou le mannitol.

4. Composition pharmaceutique solide selon la revendication 3, **caractérisée en ce que** du mannitol est présent.

5. Composition pharmaceutique solide selon la revendication 4, **caractérisée en ce que** le mannitol est présent sous forme de δ-mannitol.

6. Composition pharmaceutique solide selon l'une ou plusieurs des revendications 1 à 5, **caractérisée en ce que** le ou les alcools de sucre comprennent moins de 0,2% en poids de sucres réducteurs.

7. Composition pharmaceutique solide selon la revendication 6, **caractérisée en ce que** le ou les alcools de sucre comprennent moins de 0,05% en poids de sucres réducteurs.

8. Composition pharmaceutique solide selon l'une ou plusieurs des revendications 1 à 7, **caractérisée en ce qu'**elle se trouve sous forme de granulé, de capsule ou de comprimé.

9. Composition pharmaceutique solide selon la revendication 8, **caractérisée en ce qu'**il s'agit d'un comprimé.

10. Composition pharmaceutique solide selon la revendication 9 et/ou 10, **caractérisée en ce que** le liant présent est l'hydroxypropyl méthylcellulose.

11. Composition pharmaceutique solide selon l'une ou plusieurs des revendications 1 à 10, **caractérisée en ce qu'**elle comprend de 0,1 à 10% en poids de (R)-(-)-2-[5-(4-fluorophényl)-3-pyridylméthylaminométhyl]chromane, de 50 à 99,9% en poids de mannitol et de 1 à 5% en poids d'hydroxypropyl méthylcellulose.

12. Procédé de préparation d'une composition pharmaceutique solide selon l'une ou plusieurs des revendications 1 à 10, **caractérisé en ce qu'**elle est préparée par compression directe ou par compression de granulés produits au moyen d'une granulation par voie humide ou sèche, et est ensuite éventuellement pourvue d'un enrobage.

13. Procédé selon la revendication 12, **caractérisé en ce qu'**elle est préparée par compression de granulés produits au moyen d'une granulation par voie humide.
